# EUROPÄISCHE PATENTSCHRIFT

(11) **EP 1 324 762 B1**
(45) Veröffentlichungstag und Bekanntmachung des Hinweises auf die Patenterteilung: **23.08.2006**
(21) Anmeldenummer: 01982356.6
(22) Anmeldetag: 26.09.2001
(51) Int. Cl.: A61K 35/22, C07J 75/00, A61K 38/22

(54) **KRESOLABTRENNUNG AUS STUTENHARN**
SEPARATION OF CRESOL FROM MARE'S URINE
SEPARATION DU CRESOL A PARTIR DE L'URINE DE JUMENTS

(30) Priorität: 29.09.2000 DE 10048524
(43) Veröffentlichungstag der Anmeldung: 09.07.2003
(73) Patentinhaber: Solvay Pharmaceuticals GmbH, 30173 Hannover (DE)
(72) Erfinder: RASCHE, Heinz-Helmer, 31303 Burgdorf (DE); WILBRAND, Kirsten, 30419 Hannover (DE); WOLTMANN, Kerstin, 30457 Hannover (DE)
(74) Vertreter: Gosmann, Martin
(86) Internationale Anmeldenummer: PCT/EP2001/011115
(87) Internationale Veröffentlichungsnummer: WO 2002/026760

(56) Entgegenhaltungen:
- US-A- 5 723 454
- US-A- 5 814 624

## Beschreibung

Die vorliegende Erfindung betrifft allgemein die Gewinnung eines natürlichen Gemisches konjugierter Oestrogene aus dem Harn trächtiger Stuten und speziell die Kresolabtrennung aus Stutenharn.

Oestrogene werden in der Medizin zur Hormonsubstitutionstherapie eingesetzt. Insbesondere werden Oestrogengemische zur Behandlung und Prophylaxe der bei Frauen auftretenden Beschwerden der Wechseljahre nach natürlicher oder artifizieller Menopause eingesetzt. Hierbei haben sich natürliche Gemische konjugierter Oestrogene, wie sie im Harn trächtiger Stuten vorliegen, als besonders wirksam und gut verträglich erwiesen.

Der gelöste Feststoffgehalt im Harn trächtiger Stuten (= pregnant mares' urine, im folgenden abgekürzt als "PMU") kann natürlicherweise in weiten Bereichen schwanken und im allgemeinen in einem Bereich von 40 - 90 g Trockensubstanz pro Liter liegen. Neben Harnstoff und sonstigen üblichen Harninhaltsstoffen sind in dem Feststoffgehalt des PMU phenolische Bestandteile in Mengen von ca. 2 - 5 Gew.-% bezogen auf Trockensubstanz enthalten. Unter diesen phenolischen Bestandteilen befinden sich Kresole und das als HPMF bekannte Dihydro-3,4-bis[(3-hydroxyphenyl)methyl]-2(3H)-furanon. Diese können in freier oder konjugierter Form vorliegen. In dem PMU ist ein natürliches Gemisch von Oestrogenen enthalten, welches weitgehend in konjugierter Form, z. B. als Schwefelsäurehalbester-Natriumsalz (im folgenden abgekürzt als "Sulfatsalz"), vorliegt. Der Gehalt an konjugierten Oestrogenen (= conjugated estrogen, im folgenden abgekürzt als "CE") kann berechnet als Oestrogensulfatsalz und bezogen auf Trockensubstanz zwischen 0,3 und 1 Gew.-% betragen.

Zur direkten Aufbereitung und Gewinnung der im PMU enthaltenen konjugierten Oestrogene sind im Stand der Technik verschiedene Verfahrensweisen beschrieben. Üblicherweise werden konjugierte Oestrogene enthaltende Extrakte aus dem PMU durch Extraktion mit einem polaren mit Wasser nicht oder nur wenig mischbaren organischen Lösemittel wie beispielsweise Essigsäureethylester, n-Butanol oder Cyclohexanol gewonnen. Bei derartigen Flüssig-Flüssig-Extraktionen treten jedoch eine Vielzahl von Problemen, wie starke Schaumbildung, Sedimentbildung, Emulsionsbildung und schlechte Phasentrennung auf. Es werden im allgemeinen mehrere Extraktionsschritte benötigt, was zu Verlusten und einer nur teilweisen Gewinnung des Oestrogengehaltes führt. Zur Vermeidung dieser Nachteile wurden im Stand der Technik daher eine Reihe von Festphasen-Extraktionsverfahren vorgeschlagen.

Für die Aufbereitung von kleinen Mengen an Harn- und Plasmaflüssigkeiten für eine analytische Bestimmung von Oestrogenen mittels Gaschromatographie beschreiben Heikkinnen et al. (Clin. Chem. 27/7,(1981), 1186 - 1189) und Shackleton et al. (Clinica Chimica Acta 107(1980) 231 - 243) eine Festphasen-Extraktion von Oestrogenen mittels einer Kartusche mit Octadecylsilanreste enthaltendem silanisiertem Kieselgel (Sep-Pak^{R} C¹⁸-cartridge, Hersteller Waters Ass. Inc. Milford, MA, USA). Hierbei werden die Oestrogene mit Methanol von der Kartusche eluiert.

Von H. L. Bradlow wurde 1968 (siehe Steroids 11 (1968), 265 - 272) vorgeschlagen, zur Extraktion von konjugierten Oestrogenen aus Harn Amberlite XAD-2^{R}, ein neutrales unpolares hydrophobes Polystyrolharz der Fa. Rohm und Haas, einzusetzen. Die angegebene Adsorptionskapazität ist gering. Nach Bradlow wird ein gegebenenfalls verdünnter Harn mit einer niedrigen Durchflußgeschwindigkeit durch eine das Harz enthaltende Säule geleitet. Die Oestrogene werden mit Methanol oder Ethanol eluiert.

Neuere Patentanmeldungen beschreiben Verfahren zur Gewinnung eines, das natürliche Gemisch konjugierter Oestrogene aus Stutenharn enthaltenden Extraktes durch Festphasenextraktion des Gemisches konjugierter Oestrogene aus dem Harn trächtiger Stuten z. B. an RP-Kieselgel (WO 98/08525) oder an nichtionischen semipolaren polymeren Adsorberharzen (WO 98/08526). Die in diesen internationalen Patentanmeldungen beschriebenen Verfahren setzen noch PMU-Ausgangsmaterialien mit relativ hohen Anteilen phenolischer Harninhaltsstoffe wie z. B. Kresol und HPMF ein, die zwar durch die beschriebenen Verfahren bereits erfolgreich abgetrennt werden können, andererseits jedoch die Effizienz der eigentlichen Aufarbeitung als unerwünschte Begleitstoffe, z. B. durch Kapazitätsverminderung des jeweiligen Adsorbens, beschränken.

Je nach Herkunft des Harns bzw. Fütterung der Pferde kann auch in frischem nativen Harn ein Kresolgehalt von mehr als 500 mg/l bis gelegentlich sogar über 1.500 mg/l zu verzeichnen sein. Der Kresolgehalt erhöht sich zusätzlich je nach Herkunft, Alter des Harns, Grad der Verkeimung und den Lagerungsbedingungen, insbesondere z. B. je nach Lagerungstemperatur, durchaus auf Werte bis zu 2.000 mg/l und gegebenenfalls sogar mehr. Obwohl beispielsweise durch die alkalische Wäsche der Adsorbersäule mit nichtionischen semipolaren polymeren Adsorberharzen im Verfahren gemäß der internationalen Patentanmeldung WO 98/08526 eine sichere Kresolabtrennung gewährleistet ist, stört ein hoher Kresolgehalt aus zwei Gründen:
a) da Kresol neben den Hormonen vom Harz adsorbiert wird, sinkt die Hormonkapazität des Harzes.
b) bei hohen Kresolgehalten in der alkalischen Wäsche ist ein Hormonverlust von einigen Prozent während dieses Schrittes zu verzeichnen.

Zur Vermeidung dieser Nachteile ist es wünschenswert, Harn mit einem möglichst geringen Kresolgehalt zu verarbeiten. Da geringe Kresolgehalte im PMU aus Transportgründen - z. B. bei weltweiter Sammlung, auch in entlegenen Gegenden - oder auch bedingt durch Rasse und Fütterung der Pferde nicht gewährleistet werden können, muß nach anderen Maßnahmen gesucht werden, den Kresolgehalt im PMU vor der Aufarbeitung und Gewinnung der Gemische konjugierter Oestrogene zu minimieren. Bisherige Versuche z. B. durch Adsorption, führten nicht zum Erfolg, da mit einer Kresolreduzierung mehr oder weniger hohe, nicht akzeptierbare Hormonverluste einhergingen.

Neben der im Stand der Technik beschriebenen Optimierung der unmittelbaren, vollständigen Aufarbeitung von Harn trächtiger Stuten (PMU) zur Gewinnung natürlicher Gemische konjugierter Oestrogene (CE) sind daher auch die der Aufarbeitung vorgelagerten Schritte, wie z. B. Sicherstellung einer oestrogenschonenden Entfernung von unerwünschten Begleitstoffen wie z. B. Kresolen, von besonderer Bedeutung für eine effektive Aufarbeitung und Gewinnung eines qualitativ und quantitativ hochwertigen Gemisches konjugierter Oestrogene.

Aufgabe der vorliegenden Erfindung ist es somit, ein technisches Verfahren zur Abtrennung von Kresolen aus PMU zu entwickeln, welches ein weitgehend an Kresol abgereichertes PMU liefert. Solche weitgehend von Kresol befreiten PMU stellen ein qualitativ und quantitativ hochwertiges Ausgangsmaterial für die eigentliche Aufarbeitung zur Gewinnung des natürlichen Gemisches konjugierter Oestrogene aus dem PMU dar.

Es wurde nun ein Verfahren gefunden, das eine spezielle Art der Filtration, eine sogenannte Pervaporation, darstellt und mit welchem sich der Kresolgehalt im Harn trächtiger Stuten (PMU) einfach und effektiv vermindern läßt. Das erfindungsgemäße Verfahren zur Abtrennung von Kresol aus dem Harn trächtiger Stuten (PMU)zeichnet sich dadurch aus, daß man den Kresolgehalt einer wäßrigen Ausgangslösung von PMU durch Pervaporation mittels einer porenfreien polymeren Membran aus Silikon vermindert, indem Kresol auf die Permeatseite der Membran permeiert wird und die behandelte PMU-Lösung als ein Gemisch konjugierter Oestrogene enthaltendes Retentat mit vermindertem Kresolgehalt gewonnen wird.

Die Pervaporation ist eine spezielle Art der Filtration, bei der eine Komponente eines flüssigen Gemisches (Ausgangslösung) von der Feedseite einer porenfreien, polymeren Membran auf die Permeatseite transportiert wird. Vielfach ist damit ein Phasenwechsel von flüssig zu gasförmig (Gasraum bzw. Vakuum) verbunden, es kann aber auch ein Übergang von einer flüssigen Phase der Ausgangslösung, aus der ein bestimmter Stoff abgetrennt werden soll, in eine zweite flüssige Phase, die den abzutrennenden Stoff auf der Permeatseite aufnimmt, vorliegen. Für die restlichen Komponenten des Gemisches bzw. der Ausgangslösung stellt die Membran eine fast vollständige Barriere dar.

Ein selektiver Transport des aus einem Gemisch abzutrennenden Stoffes liegt vor, wenn die Konzentrationen dieser Gemischkomponente im Feed und in der Membran verschieden sind. Die Trennwirkung beruht auf Interaktion der permeierenden, d. h. der abzutrennenden Gemischkomponente mit der verwendeten Membran, die in der Lage ist, die abzutrennende Gemischkomponente selektiv zu sorbieren, durch die Membran zu diffundieren und auf der Permeatseite zu desorbieren. Erfindungsgemäß wurde gefunden, daß sich porenfreie polymere Membranen aus Silikon hervorragend zur Verminderung bzw. weitgehenden Abtrennung von Kresol aus dem Harn trächtiger Stuten (PMU) eignen, wobei die erwünschten, im PMU enthaltenen konjugierten Oestrogene, wie Estron und Equilin, produktschonend vollständig im Retentat verbleiben.

Im Rahmen der Erfindung können an sich beliebige Pervaporationsanlagen eingesetzt werden, die in irgendeiner Form mit einer oder mehreren porenfreien polymeren Membranen aus Silikon ausgerüstet sind.

In einer Variante des erfindungsgemäßen Verfahrens zur Abtrennung von Kresol aus dem Harn trächtiger Stuten (PMU) wird die Verminderung des Kresolgehaltes einer wäßrigen Ausgangslösung von PMU durch Pervaporation dadurch erreicht, indem man die Kresol-haltige wäßrige Ausgangslösung von PMU durch einen als porenfreie polymere Membran dienenden Silikonschlauch oder ein Silikonhohlfasermodul pumpt, wobei Kresol auf die Permeatseite permeiert wird und die behandelte PMU-Lösung als ein Gemisch konjugierter Oestrogene enthaltendes Retentat mit vermindertem Kresolgehalt gewonnen wird.

In den oben beschriebenen Varianten des erfindungsgemäßen Verfahrens kann auf der Permeatseite ein Vakuum, eine Gasphase oder eine Kresol-aufnehmende Permeatflüssigkeit vorliegen. Eine bevorzugte Ausgestaltung des erfindungsgemäßen Verfahrens zeichnet sich dadurch aus, daß auf der Permeatseite eine Permeatflüssigkeit, insbesondere Wasser oder eine wäßrige ethanolische Lösung, vorliegt. Hierbei hat es sich als vorteilhaft erwiesen, wenn man während der Pervaporation die Kresol-aufnehmende Permeatflüssigkeit in Intervallen oder kontinuierlich erneuert. Hierdurch kann ein günstiges treibendes Konzentrationsgefälle aufrechterhalten werden.

An sich können im erfindungsgemäßen Verfahren porenfreie polymere Membranen aus verschiedensten Silikon-Typen verwendet werden. Es können zweckmäßigerweise beispielsweise porenfreie polymere Membran-Silikone wie diese als technische Produkte (siehe z. B. Winnacker-Küchler (3.) 5: 252 - 286) verfügbar sind. Solche Silikone gehören zu einer umfangreichen Gruppe von synthetischen polymeren siliziumorganischen Verbindungen, in denen Siliziumatome über Sauerstoffatome verknüpft und die restlichen Valenzen des Siliziums durch Kohlenwasserstoffreste (z. B. meist Methylreste, gelegentlich auch andere Reste wie z. B. Ethyl-, Propyl- oder Phenylgruppen) abgesättigt sind. Erwähnenswert sind z. B. "Silikonkautschuke", z. B. heiß vulkanisierende Silikonkautschukmassen ("Heißkautschuk"), die meist plastisch verformbare, gerade noch fließfähige Materialien darstellen, die durch Vulkanisation ein wärmebeständiges elastisches Silikongummi ergeben, der zu Materialien für verschiedenste Anwendungen weiterverarbeitet werden kann, insbesondere z. B. zu Silikongummischläuchen, die in der chemischen Industrie und in der Medizin eingesetzt werden können.

Die jeweils konkrete Ausgestaltung des erfindungsgemäßen Verfahrens im Rahmen der im Einzelfall vorliegenden Rahmenbedingungen dürfte den Fachmann vor keine besonderen Probleme stellen. Insbesondere lassen sich die jeweils optimalen Verfahrensbedingungen durch einige wenige Vorversuche ermitteln, beispielsweise in der Art wie durch die erfindungsgemäßen Beispiele weiter unten erläutert.

Die Erfindung weist den Vorteil auf, daß durch die Kresolentfernung das spezifische Harnvolumen, das pro Liter Harz auf eine Säule zur Abtrennung und Isolierung von konjugierten Östrogenen aufgegeben werden kann, erhöht wird. Hierdurch kann bei der nachfolgenden eigentlichen Aufarbeitung von PMU an einer Harzsäule die unerwünschte Adsorption von Kresol an das Harz (z. B. an das häufig verwendete Austauscherharz XAD-7) vermieden und die zusätzlich gewonnene Kapazität für die Adsorption der wertvollen Hormonbestandteile genutzt werden. Weiterhin wirkt sich die Verminderung des Kresolgehaltes in PMU auch vorteilhaft auf eine Minimierung der Hormonverluste während der Aufarbeitung des PMU, z. B. bei alkalischer Säulenwäsche (siehe z. B. WO 98/08526), aus.

### Figurenbeschreibung

- **Fig. 1:**: Versuchsaufbauskizze zur flüssig-flüssig-Extraktion von Kresol aus PMU mittels Pervaporation durch Silikonschlauch als porenfreie polymere Membran.
- **Fig. 2:**: Versuchsaufbauskizze zur flüssig-flüssig-Extraktion von Kresol am PMU mittels Pervaporation unter Verwendung eines Silikonhohlfasermoduls.

### Beispiele

Die nachfolgenden Beispiele sollen die Erfindung näher erläutern, ohne jedoch ihren Umfang zu beschränken.

### Prinzip der Pervaporation

Die Pervaporation ist eine spezielle Art der Filtration, bei der eine Komponente eines flüssigen Gemisches (Ausgangslösung) von der Feedseite einer porenfreien, polymeren Membran auf die Permeatseite transportiert wird. Vielfach ist damit ein Phasenwechsel von flüssig zu gasförmig (Gasraum bzw. Vakuum) verbunden, es kann aber auch ein Übergang von einer flüssigen Phase der Ausgangslösung, aus der ein bestimmter Stoff abgetrennt werden soll, in eine zweite flüssige Phase, die den abzutrennenden Stoff auf der Permeatseite aufnimmt, vorliegen. Für die restlichen Komponenten des Gemisches bzw. der Ausgangslösung stellt die Membran eine fast vollständige Barriere dar.

Ein selektiver Transport liegt vor, wenn die Konzentrationen der Gemischkomponenten im Feed und in der Membran verschieden sind. Die Trennwirkung beruht auf Interaktion der permeierenden, d. h. der abzutrennenden Gemischkomponente mit der verwendeten Membran, die in der Lage ist, die abzutrennende Gemischkomponente selektiv zu sorbieren, durch die Membran zu diffundieren und auf der Permeatseite zu desorbieren.

In den nachfolgenden Versuchen des Beispiels 1 wurde eine Pervaporationsanlage verwendet, auf deren Permeatseite ein Flüssigkeitsstrom angeboten wurde. Das Verfahren gemäß Beispiel 1 ist nicht einschränkend zu verstehen, da auch in Pervaporationsanlagen gearbeitet werden kann, die auf der Permeatseite einen Gasraum bzw. Vakuum zur Aufnahme des abzutrennenden Stoffes anbieten. Eine andere Verfahrensweise, unter Verwendung eines Hohlfasermoduls, die ebenfalls nicht einschränkend zu verstehen ist, findet sich in Beispiel 3.

### Beispiel 1:

### Pervaporationsversuche (flüssig-flüssig, Silikonschlauch)

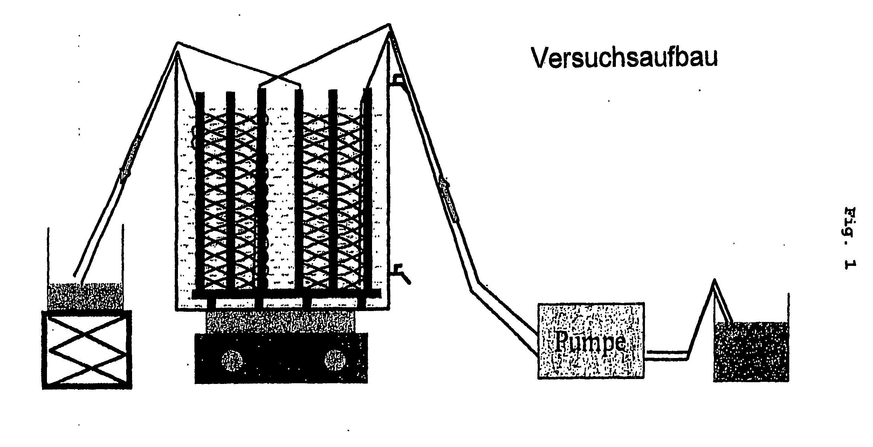

In den nachfolgenden Pervaporationsversuchen dieses Beispiels wurde eine aus Schläuchen (Membran) und umgebender Flüssigkeitsstrom (Permeatseite) gebildeter Versuchsaufbau verwendet. Der Versuchsaufbau ist in Fig. 1 skizziert. Da es wünschenswert ist, von einer spezifisch großen Fläche der Membran auszugehen, fanden Schläuche mit einem Innendurchmesser von ca. 1 mm und einer Wandstärke von ca. 0,4 mm Anwendung.

### Versuch 1:

Aus einer Vorlage wurde Harn mit einem Volumen von 30 ml/h durch einen Silikonschlauch mit einer Länge von 25 m gepumpt. Der Schlauch lag aufgerollt in einer Schale mit 1 1 30%-ethanolischer Lösung. Die Analyse ergab, daß im Harn nur Kresol abgereichert wird und in die ethanolische Lösung (Permeat) diffundiert.

**Tabelle 1: Ergebnisse Versuch 1**

| | **Estron (mg/l)** | **Equilin (mg/l)** | **Kresol (mg/l)** | **HPMF (mg/l)** |
|---|---|---|---|---|
| **Harn-Ausganglösung** | 41,5 | 15,8 | 89,4 | 1,2 |
| **Schlauch-Austritt nach 1 h (Retentat)** | 41,4 | 16,0 | 15,5 | 1,5 |
| **Schlauch-Austritt nach 4 h (Retentat)** | 41,4 | 15,9 | 10,9 | 1,5 |
| **Ethanolische Lösung nach 4 h (Permeat)** | 0 | 0 | 9,8 | 0 |

### Versuch 2:

Versuchsdurchführung wie zuvor im Versuch 1, lediglich die ethanolische Lösung wurde durch VE-Wasser (VE = vollentmineralisiertes Wasser) ersetzt. Die Analyse ergab im Prinzip das gleiche Bild wie im Versuch 1. Auch in diesem Versuch diffundierte Kresol in die als Permeat dienende Wasserphase. Es traten keine Verluste an Estron und Equilin auf.

**Tabelle 2: Ergebnisse Versuch 2**

| | **Estron (mg/l)** | **Equilin (mg/l)** | **Kresol (mg/l)** | **HPMF (mg/l)** |
|---|---|---|---|---|
| **Harn-Ausganglösung** | 41,5 | 15,8 | 89,4 | 1,2 |
| **Schlauch-Austritt nach 7 h (Retentat)** | 42,8 | 16,4 | 16,0 | 1,3 |
| **Wasserphase nach 7 h (Permeat)** | 0 | 0 | 16,2 | 0 |

### Versuch 3:

Die Versuchsanordnung der Versuche 1 und 2 wurde auf vier parallele Schläuche je 25 m erweitert. Der Ausgangsharn weist einen hohen Kresolgehalt auf. Der Versuchsaufbau ist beispielhaft in Fig. 1 illustriert. Je 25 m Silikonschlauch (Innendurchmesser 1 mm, Wandstärke 0,4 mm) wurden auf den 4 Verstrebungen aufgewickelt und diese dann in einem Behälter untergebracht. Jeder der 4 Schläuche wurde einzeln an eine Schlauchpumpe angeschlossen. Auf der Permeatseite, d. h. auf der Außenseite um die Schläuche herum befanden sich 8 1 einer wäßrigen Ethanollösung, die gerührt wurden. Danach wurde die PMU-Ausgangslösung mit 8 RPM (= 15,6 ml/h) durch die Schläuche gepumpt. Pro Schlauch wurde jeweils für ein Zeitintervall von 2 h das Retentat (= behandeltes PMU) aufgefangen, und die Konzentrationen der Komponenten dieser Fraktionen bestimmt. Nach 6 h , d. h. nach der dritten Fraktion wurde der Versuch beendet. Gesamtmenge an PMU, die durch alle 4 Schläuche geleitet wurde betrug ca. 374 ml in 6 h, was insgesamt 232 mg Kresol entspricht. Die Gesamtmenge Kresol nach 6 h in 8 1 wäßrigem Permeat betrug 307 mg Kresol.

Die Analyse der Komponenten in Retentat und Permeat ergab das gleiche Bild wie zuvor in den Versuchen 1 und 2 erhalten: Estron und Equilin wurden zurückgehalten, Kresol zu 95 % abgetrennt.

**Tabelle 3:**

| Ergebnisse des Versuchs 3; Kresol-Entfernung durch flüssigflüssig Extraktion mittels Silikonschlauch (Pervaporation) | | | | | | |
|---|---|---|---|---|---|---|
| **Probe** | **TS Gehalt** | **pH-Wert** | **Equilin** | **Estron [mg/l]** | **Kresol [mg/l]** | **HPMF [mg/l]** |
| **Nullprobe** | 5,82 | 8,04 | 25,7 | 17,2 | 620,2 | 129,3 |
| **Schlauch 1, 2h** | 5,81 | 8,63 | 26,8 | 17, 6 | 32,2 | 110,5 |
| **Schlauch 2, 2h** | 5,87 | 8,63 | 27,8 | 17,8 | 35,6 | 111,2 |
| **Schlauch 3, 2h** | 5,78 | 8,65 | 28,5 | 18,6 | 32,2 | 109,7 |
| **Schlauch 4, 2h** | 5,78 | 8,69 | 28,0 | 18,4 | 37,1 | 108,3 |
| **Schlauch 1, 4h** | 5,91 | 8,62 | 28,1 | 18,1 | 36,4 | 112,4 |
| **Schlauch 2, 4h** | 5,82 | 8,61 | 28,4 | 19,8 | 36,2 | 109,1 |
| **Schlauch 3, 4h** | 5,82 | 8,61 | 26,3 | 18,7 | 32,5 | 115,0 |
| **Schlauch 4, 4h** | 5,79 | 8,61 | 27,7 | 18,4 | 35,8 | 111,4 |
| **Schlauch 1, 6h** | 5,81 | 8,59 | 28,0 | 18,5 | 46,1 | 111,5 |
| **Schlauch 2, 6h** | 5,73 | 8,59 | 28,8 | 17,8 | 45,2 | 110,3 |
| **Schlauch 3, 6h** | 5,76 | 8,59 | 28,3 | 19,9 | 44,4 | 114,7 |
| **Schlauch 4, 6h** | 5,89 | 8,59 | 28,8 | 19,3 | 47,7 | 111,7 |
| **Permeat, Versuchsende** | 0,00 | 5,55 | 0,0 | 0,0 | 38,4 | 0,0 |

Der Anstieg der Kresolwerte in den einzelnen Schläuchen nach 2 h bzw. 4 h beruht wahrscheinlich auf einer Kresolanreicherung in der aufnehmenden Wasserphase. Dadurch bedingt verringert sich das die Permeation treibende Konzentrationsgefälle. Gewünschtenfalls bietet sich zum Gegensteuern eine Vergrößerung des Volumens bzw. ein häufigerer Austausch des Permeats an.

### Ergebnis der Versuche 1 bis 3:

Die im Beispiel 1 untersuchte, porenfreien Membran aus Silikon eignet sich zur selektiven Abtrennung von Kresol aus Stutenharn. Estron und Equilin werden quantitativ zurückgehalten. Grundsätzlich steht hiermit ein Verfahren zur Verfügung, um hohe Kresolgehalte in PMU verschiedenster Herkunft zu reduzieren und dadurch die Hormonkapazität von Adsorbersäulen bei der nachfolgenden Aufarbeitung des PMU zur Gewinnung von Gemischen konjugierter Oestrogene zu erhöhen und die sonst auftretenden Waschverluste von konjugierten Oestrogenen zu minimieren.

### Beispiel 2:

### Vergleichsbeispiel

Von fünf untersuchten, porenfreien Membranen eignete sich nur Silikon zur selektiven Abtrennung von Kresol aus Stutenharn, bei der Estron und Equilin quantitativ zurückgehalten wurden.

In ersten Versuchen wurden neben Silikon analog zum Beispiel 1 (Versuche 1 und 2) auch nachstehende Materialien in Form von Schläuchen untersucht: Polyethylen (PE), Polyvinylchlorid (PVC), Cellulose (2 Versuche).

Ohne auf weitere Einzelheiten einzugehen, ergab sich für diese Materialien folgendes Ergebnis: Bei PE und PVC fand keine Diffusion von Kresol, HPMF und Estron statt. Bei den zwei untersuchten, unterschiedlichen Cellulose-Dialyseschläuchen diffundierten teilweise Kresol und Estron, nicht jedoch HPMF. Lediglich Silikonschläuche ließen, wie im Beispiel 1 gezeigt, selektiv nur Kresol passieren.

### Beispiel 3:

### Pervaporationsversuche (Silikonhohlfasermodul)

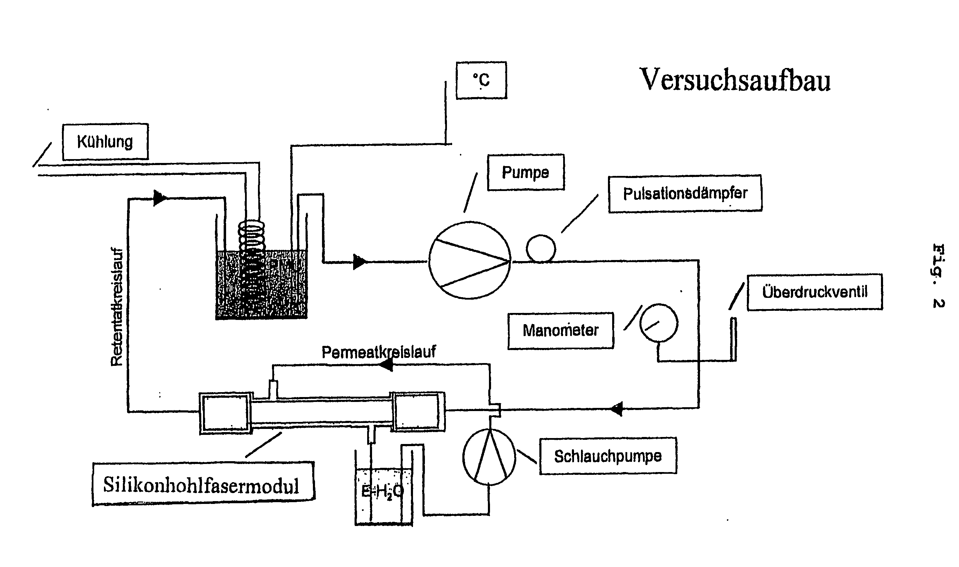

Im Pervaporationsversuch dieses Beispiels wurde eine Anlage mit einem Silikonkohlfasermodul eingesetzt: Länge 30 cm, 100 Kapillaren mit Innendurchmesser 0,005 m. Es stand eine Pervaporationsfläche von insgesamt 0,09425 m² zur Verfügung. Der Versuchsaufbau ist in Fig. 2 skizziert. Der Versuch wurde bei einer Temperatur von 19 °C unter analogen Bedingungen zum Beispiel 1 gefahren, und die Probenentnahme erfolgte nach 3 Stunden. Die Ergebnisse der Kresolentfernung aus PMU mit dem Silikonkohlfasermodul sind in der nachfolgenden Tabelle 4 wiedergegeben.

**Tabelle 4: Ergebnisse Beispiel 3**

| | **Estron (mg/l)** | **Equilin (mg/l)** | **Kresol (mg/l)** | **HPMF (mg/l)** |
|---|---|---|---|---|
| **Harn-Ausgangslösung** | 13,0 | 27,4 | 770,8 | 12,5 |
| **Retentat nach 3 h** | 12,6 | 26,9 | 545,5 | 11,7 |
| **Permeat nach 3 h** | 0,0 | 0,0 | 8,9 | 0,0 |

## Patentansprüche

1. Verfahren zur Abtrennung von Kresol aus dem Harn trächtiger Stuten (PMU), **dadurch gekennzeichnet, daß** man den Kresolgehalt einer wäßrigen Ausgangslösung von PMU durch Pervaporation mittels einer porenfreien polymere Membran aus Silikon vermindert, indem Kresol auf die Permeatseite der Membran permeiert wird und die behandelte PMU-Lösung als ein Gemisch konjugierter Oestrogene enthaltendes Retentat mit vermindertem Kresolgehalt gewonnen wird.

2. Verfahren zur Abtrennung von Kresol aus dem Harn trächtiger Stuten (PMU) nach Anspruch 1, **dadurch gekennzeichnet, daß** man den Kresolgehalt einer wäßrigen Ausgangslösung von PMU durch Pervaporation vermindert, indem man die Kresol-haltige wäßrige Ausgangslösung von PMU durch einen als porenfreie polymere Membran dienenden Silikonschlauch oder ein Silikonhohlfasermodul pumpt, wobei Kresol auf die Permeatseite permeiert wird und die behandelte PMU-Lösung als ein Gemisch konjugierter Oestrogene enthaltendes Retentat mit vermindertem Kresolgehalt gewonnen wird.

3. Verfahren nach einem der Ansprüche 1 oder 2, **dadurch gekennzeichnet, daß** auf der Permeatseite ein Vakuum, eine Gasphase oder eine Kresol-aufnehmende Permeatflüssigkeit vorliegt.

4. Verfahren nach Anspruch 3, **dadurch gekennzeichnet, daß** auf der Permeatseite eine Permeatflüssigkeit, vorzugsweise Wasser oder eine wäßrige ethanolische Lösung, vorliegt.

5. Verfahren nach Anspruch 4, **dadurch gekennzeichnet, daß** man während der Pervaporation die Kresol-aufnehmende Permeatflüssigkeit in Intervallen oder kontinuierlich erneuert.

## Claims

1. A method for the separation of cresol from pregnant mares' urine (PMU), **characterised in that** the cresol content of an aqueous starting solution of PMU is reduced by pervaporation by means of a pore-free polymeric silicone membrane, by permeating cresol on to the permeate side of the membrane and obtaining the treated PMU solution as retentate with reduced cresol content containing a mixture of conjugated oestrogens.

2. A method for the separation of cresol from pregnant mares' urine (PMU) according to Claim 1, **characterised in that** the cresol content of an aqueous starting solution of PMU is reduced by pervaporation, by pumping the cresol-containing aqueous starting solution of PMU through a silicone tube acting as a pore-free polymeric membrane or a silicone hollow fibre module, with cresol being permeated on to the permeate side and the treated PMU solution being obtained as a retentate with reduced cresol content containing a mixture of conjugated oestrogens.

3. A method according to one of Claims 1 or 2, **characterised in that** a vacuum, a gas phase or a cresol-receiving permeate liquid is present on the permeate side.

4. A method according to Claim 3, **characterised in that** a permeate liquid, preferably water or an aqueous ethanolic solution, is present on the permeate side.

5. A method according to Claim 4, **characterised in that** the cresol-receiving permeate liquid is replenished at intervals or continuously during the pervaporation.

## Revendications

1. Procédé de séparation de crésol à partir d'urine de juments gravides (PMU, Pregnant Mare Urine), **caractérisé en ce que** l'on réduit par pervaporation la teneur en crésol d'une solution de départ aqueuse de PMU au moyen d'une membrane polymère en silicone sans porosité, en faisant traverser le crésol côté perméat de la membrane et en recueillant la solution de PMU traitée sous la forme d'un rétentat contenant un mélange d'oestrogènes conjugués ayant une teneur en crésol réduite.

2. Procédé de séparation de crésol à partir d'urine de juments gravides (PMU) selon la revendication 1, **caractérisé en ce que** l'on réduit par pervaporation la teneur en crésol d'une solution de départ aqueuse de PMU, en pompant la solution de départ aqueuse de PMU contenant du crésol à travers un tuyau flexible de silicone servant de membrane polymère sans porosité ou à travers un module de fibres creuses siliconées, le créasol traversant côté perméat et la solution de PMU traitée étant recueillie sous la forme d'un rétentat contenant un mélange d'oestrogènes conjugués ayant une teneur en crésol réduite.

3. Procédé selon la revendication 1 ou 2, **caractérisé en ce qu'**un vide, une phase gazeuse ou un liquide perméat absorbant le crésol est présent côté perméat.

4. Procédé selon la revendication 3, **caractérisé en ce qu'**un liquide perméat, de préférence de l'eau ou une solution d'éthanol aqueuse, est présent côté perméat.

5. Procédé selon la revendication 4, **caractérisé en ce que** le liquide perméat absorbant le crésol est renouvelé par intervalles ou en continu au cours de la pervaporation.
